# EUROPEAN PATENT SPECIFICATION

(11) **EP 4 385 045 B1**
(45) Date of publication and mention of the grant of the patent: **01.10.2025**
(21) Application number: 22765068.6
(22) Date of filing: 11.08.2022
(51) Int. Cl.: G21G 1/00

(54) **SYSTEM AND METHOD OF THE PRODUCTION AND ISOLATION OF CHARGED RADIONUCLIDES**
SYSTEM UND VERFAHREN ZUR HERSTELLUNG UND ISOLIERUNG VON GELADENEN RADIONUKLIDEN
SYSTÈME ET PROCÉDÉ DE PRODUCTION ET D'ISOLATION DE RADIONUCLÉIDES CHARGÉS

(30) Priority: 11.08.2021 EP 21190882
(43) Date of publication of application: 19.06.2024
(73) Proprietor: NRG PALLAS B.V., 1755 LE Petten (NL)
(72) Inventor: NIEUWLAND, Pieter Jos, 1755 ZG PETTEN PETTEN (NL); VAN DER BORN, Dion, 1755 ZG PETTEN (NL); ZWAAGSTRA, Oene, 1755 ZG PETTEN (NL); BAKKER, Klaas, 1755 ZG PETTEN (NL); BUCHATSKAYA, Yulia, 1755 ZG PETTEN (NL); DE GROOT, Sander, 1755 ZG PETTEN (NL)
(74) Representative: EP&C
(86) International application number: PCT/EP2022/072556
(87) International publication number: WO 2023/017122

(56) References cited:
- RU-C1- 2 742 138
- US-A- 5 038 046
- BARANYAI A ET AL: "212Bi-212Po alpha source for the calibration and functional testing of silicon detectors: Preparation and characterisation", APPLIED RADIATION AND ISOTOPES, ELSEVIER, OXFORD, GB, vol. 158, 11 January 2020 (2020-01-11), XP086080311, ISSN: 0969-8043, [retrieved on 20200111], DOI: 10.1016/J.APRADISO.2019.109028

## Description

### Field of the invention

The invention relates to a method and apparatus for the production of radionuclides formed by the decay of radioactive gas following by capturing of charged daughter radionuclides using an electric field. The present invention further relates to a method for the production of radionuclides for use in therapeutic applications.

### Background of the invention

In targeted alpha therapy (TAT), an alpha-emitting radionuclide is attached to a compound adhering to (receptors of) specific cancer cells, thereby delivering a (fatal) dose of alpha-radiation to the cancer cells, leaving healthy cells largely unaffected. The perspective of this method is an effective treatment without major side effects, by a targeted therapeutic approach. Beta- emitters such as Lutetium 177 are already showing a large perspective for these type of targeted therapy applications, but alpha emitters hold the promise of higher therapeutic efficiency, either used as the single radiation damage providing radionuclide, or in combination with other beta- or Auger emitting radionuclides or other types of therapy. Different alpha-emitting radionuclides can be foreseen for being used for TAT, for example Ac-225, At-211, Th-227, Ra-223 and more, depending on the targeting compound characteristics and requirements, such as chemical binding of the radionuclide to the compound, half-life, decay chain characteristics and alpha-damage profile. In addition, availability, affordability, ease-of-use and manufacturability determine which alpha-emitting radionuclide will be widely implemented in clinics.

TAT, in which the α-particle emitting radionuclide is specifically directed to a biological target, is gaining more attention to treat cancers as new targets are validated. Bio-vectors such as monoclonal antibodies are able to selectively transport alpha emitters to these cells. TAT has the potential for an improved therapeutic ratio over beta- emitter targeted conjugate therapy. The short path length and the intense ionization path generated by the alpha particles render alpha-emitters suitable for effective treatment of tumour tissue, including micrometastases or residual tumour after surgical debulking. Pb-212 is the longer-lived parent radionuclide of Bi-212 and serves as an in vivo generator of Bi-212. Pb-212 has demonstrated significant utility in both in vitro and in vivo models. Recent evaluation of Pb-212-TCMC-trastuzumab in a Phase I clinical trial has demonstrated the feasibility of Pb-212 in TAT for the treatment of ovarian cancer patients. The current invention thus highlights progress in radionuclide production, radiolabelling chemistry, molecular mechanisms, and application of Pb-212 to targeted pre-clinical and clinical radiation therapy for the management and treatment of cancer.

Pb-212 has specific characteristics, that can make it a very suitable radionuclide for some alpha-therapies. Pb-212 itself is a beta emitter with a half-life just over 10 hours, and has one alpha emitter in the decay chain. This can have benefit for therapies in which the targeting compound leaves the tumour location relative quickly. For these cases the long half-life of for example the currently quite popular radionuclide Ac-225, can cause insufficient or off-target alpha radiation damage. The single alpha in the Pb-212 decay chain, also avoids off-target effects in absence of multiple alpha-emissions, which occur in the decay chain of other alpha-emitting radionuclides, as alpha-emission generally leads to dissociation of the radionuclide from the targeting compound. These dissociated parent radionuclide's daughters can be subsequently distributed in the patient, which can lead to off-target damage by subsequent alpha-emissions of the radionuclide daughters.

The difficulty to produce alpha-emitters in general, and the short half-life of the Pb-212 decay chain however bring challenges with regards to establishing a suitable and economic supply chain.

To enable reliable production, with minimised transport times to avoid unnecessary decay of Pb-212 , a generator approach is used, using the Ra-224 and Th-228 parent radionuclides of Pb-212. The Th-228 is an 1.9 year half-life radionuclide that can for example be extracted from Th-232, or extracted from neutron irradiated Ra-226. Th-228 can be used as a generator continuously producing Ra-224, an radionuclide with an approximately 3.5 day half-life. Ra-224 can then subsequently be used as a generator to produce Pb-212, locally, near the patient or a central production location in the vicinity of hospitals.

The known production route for Pb-212 uses common column separation technology, well known in the actinide separation chemistry, and is for example described in patents EP3474904, WO2017220767 and WO2017093069.

EP2854870 describes a method wherein Pb-212 is formed by the decay of Ra-224 which is bound to a solid medium and is isolated by extraction of Pb-212 followed by multiple chromatographic separations. WO2017220767 describes the generation of Pb212 labelled proteins using gel-filtration chromatography. WO2017093069 describes a method to obtain Pb-212 from a solid medium via a series of washing and elution steps over chromatography columns.

The disadvantage of these routes is the elaborate double generator approach.

The regular transportation of the Ra-224 generator is cumbersome as its use is limited to about a week, and therefore requires frequent replacement. Further disadvantages are in losses due to regular elution of Th-228 from its column as it cannot be held on the column for prolonged amounts of time, as the column will deteriorate specifically due to the (alpha-) radiation damage. Regular column replacement and subsequent product losses are therefore inevitable. Column deterioration due to (alpha-)radiation damage also prevents the use of generators based on Th-228-loaded columns, which would be preferable to limit frequent Ra-224 transports.

Other methods for the generation of Pb-212 are described for instance in US5038046, which describes a method and generator for Pb-212 via the decay of Rn-220 that emanates from Th-228 . Collection of Pb-212 is done in an porous absorption body that is water soluble. Eiswirth et al. J. of Chem. Education, 1982, 59, 608 reported on Rutherfords 'thorium cow', using electrostatic radionuclide separation using a Pt-electrode from which the deposited Pb-212 was dissolved. US2018/00847474 describes methodology for the generation of daughter radionuclides such as Pb-212 by radioactive decay of gaseous Rn-220 by capturing the Rn-220 via cryogenic cooling on a column packed with a soluble salt, allowing the Rn-220 to decay into Pb-212. The dissolution of the salt then allowed capturing the desired Pb-212 radionuclide.

Deposition induced by an electric field has been described by Shinagawa et al., J. Nucl. Sci. Tech., 1968, 408; Shinagawa, J. Nucl. Sci. Tech., 1977, 163 has been described wherein a Th-228 source is used to produce Pb-212 in the gas phase and collected on a grid. Takemi et al., J. Nucl. Sci. Tech., 1973, 155; J. Nucl. Sci. Tech., 1976, 270 describes the gaseous electrodeposition of daughter radionuclides such as Pb-212 from parent radionuclides by using a three- electrode set up and by collection of the Pb-212 on a stainless steel grid. Hassfjell et al. Applied Radiation and Isotopes 2001, 45, 1021-1025 had described a Pb-212 generator wherein Rn-220 is generated from Th-228 barium stearate. The decay products, Po-216 and Pb-212 are allowed to deposit on the walls of a polyethylene bottle and are subsequently washed off with an acidic solution. Hassfjel et al., Applied Radiation and Isotopes 2001, 55, 433-439 has described a process and apparatus for the generation of Pb-212 wherein Th-228 was allowed to decay into Rn-220. The radon was bubbled through a solution of an organic solvent (hexane/methanol). The Pb-212 formed was collected in the organic solvent and on the glass surface of the bubbler and was washed off with an acidic solution. All these methods require that the desired radionuclide is to be collected from an electrode or a surface, leading to additional handling, potential Pb-212 losses and potential contamination by the electrode or the surface.

One of the disadvantages of the electrostatic capturing of charged radionuclides on electrodes or surfaces is found in the recoil effect. In the radioactive decay path from Th-228 or Ra-224, Rn-220 and its subsequent daughter radionuclides Po-216 and Pb-212 are formed by the subsequent emission of alpha particles. The energies of the emitted alpha particles are such that due to the recoil effect of the emission, the daughter radionuclides can be embedded in the source material. This problem of recoil has been partly solved in US2018/00847474by providing a gas flow or bubbling in solution. However, the decay of Rn-220 to Po-216 as well as the decay of Po-216 into Pb-212 also involves alpha decay. The use of electrostatic capture of Po-216 causes the ionised Po-216 to deposit on the electrode and part of the Pb-212 to become embedded in the electrodes of the electrostatic capturing device after the Po-216 decay, due to the recoil effect. This reduces yield and efficiency considerable.

A further example of a radioisotope preparation apparatus can be found in Baranyai et al: 212Bi-212Po alpha source for the calibration and functional testing of silicon detectors: Preparation and characterisation, Appl. Radiat. Isotopes, 158, 2020.

It is desirable to provide further improvements to the existing technology in this field and provide an apparatus and a method that is capable of producing Pb-212 radionuclides for medical use at an industrial or hospital scale that provides one or more of:
(1) Pb-212 in higher yields;
(2) Pb-212 having a radiological purity at least equal to 99.95%;
(3) Pb-212 having greater chemical purity than that of Pb-212 produced by the methods of the current state of the art;
(4) Pb-212 more quickly than the methods of the state of the art, given its relative short half-life;
(5) Pb-212 production in an automated manner;
(6) Pb-212 production in which the number of manual operations is reduced to a minimum
(7) Pb-212 production in a closed system in order to limit the risk of contaminating staff in charge of this production;
(8) Pb-212 production in a minimized amount of process steps required and avoiding the use of chromatography columns;
(9) Pb-212 production during a maximized use time of the apparatus, to avoid regular transport and replacement or reloading of the apparatus.

### Summary of the invention

The present inventors have found a generic solution for the generation, capture and isolation of charged radionuclides generated from a gaseous source. By generating a radioactive gas and allowing it to decay into charged radionuclides and application of an electric field, the ions are be selectively attracted to one of the electrodes. By placing a liquid in the line of flight of the charged radionuclides towards the electrodes, the charged radionuclides are captured in the liquid. The resulting solution contains the charged radionuclides immediately, largely avoiding direct deposition of the charged radionuclides and subsequent embedding by decay and recoil on the electrode surface.

Thus, in one aspect the invention pertains to a method for the generation of radionuclides comprising the steps of
a. providing a radioactive gas,
b. allowing the radioactive gas to decay to form charged daughter radionuclides,
c. providing a liquid in contact with the gas,
d. subjecting the charged daughter radionuclides (16) to an electric field between two electrodes, thereby absorbing the charged daughter radionuclides (16) in the liquid, and
e. isolating the liquid comprising the charged daughter radionuclides (16).

In one aspect, the invention pertains to an apparatus for the preparation and isolation of radionuclides comprising an emanation vessel (1) and a collection vessel (3), wherein the emanation vessel (1) is connected to the collection vessel (3) or wherein the emanation vessel (1) and collection vessel (3) are comprised in a singular vessel,
a. wherein the emanation vessel (1) comprises
   i. a radioactive gas emanating source material (2),
   ii. optionally a gas inlet (4),
b. wherein the collection vessel (3) contains
   i. a first electrode (5) connected to an electric source (8),
   ii. a second electrode (6) connected to the electric source (8),
   iii. a liquid section (7),
   iv. optionally a gas outlet (11).

### Brief description of the drawings:

**Figure** 1 is a schematic representation of an embodiment of the invention wherein the emanation vessel (1) and the collection vessel (3) are one body.
**Figure** 2 is a schematic representation of an embodiment of the invention wherein the emanation vessel (1) and the collection vessel (3) are separate bodies connected via a connection (15) and the radioactive gas source is provided by a radioactive source (2) in a fluid.
**Figure** 3 is a schematic representation of an embodiment of the invention wherein the emanation vessel (1) and the collection vessel (3) are separate bodies connected via a connection (15), and the radioactive gas source is provided by a solid radioactive source (2).
**Figure 4** is a schematic representation of an embodiment of the invention wherein the emanation vessel (1) and the collection vessel (3) are separate bodies connected via a connection (15), and the radioactive gas source is provided by a solid or fluid radioactive source (2) and the gas flow is bubbled through the liquid (7A) to capture the charged daughter ions (16).
**Figure 5** is a schematic representation of an embodiment of a part of the invention wherein the electrode (6) is provided under the liquid section (7) and outside the collection vessel (3) (Fig 5A), in the liquid section (7) (Fig 5B) or in (or embedded in) the outside wall of the collection vessel (3) collecting the liquid (7A) (Fig 5C).

### Detailed description of the Invention:

The invention thus pertains to a method for the generation of radionuclides comprising the steps of:
a. providing a radioactive gas,
b. allowing the radioactive gas to decay to form charged daughter radionuclides (16),
c. providing a liquid (7A) in contact with the radioactive gas,
d. subjecting the charged daughter radionuclides (16) to an electric field between two electrodes (5,6) thereby absorbing the charged daughter radionuclides (16) in the liquid (7A), and
e. isolating the liquid (7A) comprising the charged daughter radionuclides (16).

One of the advantages of the present invention resides in the provision of a method and an apparatus (generator) for charged daughter radionuclides (16) without using chromatographic separation using column materials to obtain the desired products. The method leads to the provision of high purity daughter radionuclides (16). The method leads to the provision of the daughter radionuclides (16) directly (without any further steps) in a liquid (7A) that is suitable for further isolation of the daughter radionuclides (16) from the liquid (7A) or its direct use. This is beneficial from a waste and material loss point of view. Further benefit is that column deterioration is avoided which allows the generator to be used for prolonged periods of time. This is further enhanced by the using of parent radionuclides with longer half-lives. The electric capturing in a liquid (7A) of the present invention is capable of rendering a high purity product without risk of column material contamination.

The charged radionuclides (16) can be retrieved from the radioactive gas from which they are formed by providing an electric field in the gas flow. The electric field has sufficient strength thereby forcing the charged daughter radionuclides (16) to deposit on the oppositely charged electrode of the electric field generator. In a preferred embodiment, aimed at the capture of positively charged radionuclides, the positive pole encloses the gas flow, repels the positively charged radionuclides and guides the positively charged radionuclides to the negative pole, avoiding deposition by positive-positive electric repulsion. By providing the negative pole in solution, the charged radionuclides are captured in solution and not on the electrode. After a period of decay of the radioactive gas, depending on the half time of the radioactive gas, and capturing the daughter radionuclide (16) in the liquid (7A), the daughter radionuclide captured in the liquid (7A) can be retrieved by isolating the liquid (7A) and isolating the daughter radionuclide form the liquid (7A) or processing the daughter radionuclide (16) towards applications such as its use a radiotherapeutic.

The radioactive gas can be provided as a gas or can be generated by providing a gas generator such as a radioactive source material (2) that decays into a radioactive gas. Depending on the flow of the radioactive gas, a carrier gas may be used to generate a gas flow that provides sufficient flow for the charged daughter radionuclides (16) of the radioactive gas to be absorbed (captured) in the liquid (7A).

The liquid (7A) wherein the charged daughter radionuclides (16) are captured is preferably provided in an amount that is enough to shield or immerse the electrode from direct contact with the gas containing the charged daughter radionuclides (16). By capturing the charged daughter radionuclides (16) in this manner, the attracted charged daughter radionuclides (16) are captured in the liquid (7A), their kinetic energy is reduced and they have a lower chance of becoming attached to the electrode. Also subsequent radioactive decay of charged daughter radionuclides (16) in the liquid (7A) reduces the chance of radionuclides becoming attached to or embedded in the electrode.

The source of the radioactive gas (the emanation source) can be a thorium (Th), Uranium (U) and/or a radium (Ra) radionuclide or another radionuclide that has a daughter radionuclide in the decay chain that is in gaseous form. The source of the radioactive gas can be provided as a single (point) source element, but it is preferred in embodiments to provide the source as a solution, a powder, a foam, a foil, a sponge, a salt or on a carrier. By providing the source of the radioactive gas in finely dispersed or dissolved form, the emanating gas is liberated more easily and there is lesser risk of a recoil effect that embeds the daughter radionuclide in the source material (2), lowering the yield.

The radioactive gas can be effectively removed from the emanation source by providing a gas flow that carries the radioactive gas. Thus, in embodiments, the radioactive gas is provided in a (carrier) gas flow.

For instance, in an embodiment aimed at the generation of Pb-212 radionuclides, the Ra-224 or Th-228 source material is provided and the natural decay chain will allow the radioactive gas to be formed. In a preferred embodiment, the radioactive gas is radon, preferably radon-220 (half-life 51 seconds). The Rn-220 (Radon) decays into Po-216 (Polonium) and Pb-212 (Lead) charged radionuclides. The decay takes place in gas flow created by the radon and an optional carrier gas, and leads to loss of electrons, thereby generating positively charged Polonium and Lead ions. The half-life of Po-216 is 0.15 seconds. Once formed for the radon gas and within reach of the electric field, the Po-216 charged radionuclide will move towards the electrode provided in the liquid, possibly in microseconds. The Po-216 charged radionuclide will decay into Pb-212. This decay to Pb-212 is predominantly in the liquid (7A). The Pb-212 formed may move towards the electrode but is less prone to become embedded in the electrode since the recoil effect of the decay is predominantly in the liquid (7A) and not or to a lesser extent on the surface of the electrode, especially when compared to prior art techniques discussed herein above.

In an alternative embodiment, the source material that can emanate the radioactive gas can be Ra-226. The use of irradiated Ra-226 avoids a purification step to obtain Th-228 and can operate via the decay of Th-228 through Rn-220 (t1/2= 55sec) isotope to Pb212. Directly using irradiated Ra-226 may cause the presence of Rn-219 (t1/2=4 sec) and Rn-222 (t1/2=3.82 days), which may cause impurities in the Pb-212. By optimizing the characteristics of the processing equipment (e.g. flow rate, filters and collection volume) the Pb-212 will be mainly be captured in the collection volume while the Pb-impurities (e.g. Pb-214) will not decay in the collection volume. This allows obtaining high purity Pb-212.

In embodiments, alternative radioactive sources or combined radioactive sources of a radioactive gas, such as Ra-226, can be used. Irradiation of Ra-226 to obtained Th-228 also results in the formation of Th-227 (via Ac-227). Th-227 can decay into (gaseous) Rn-219 (t1/2=4 seconds). This effect can be reduced by holding the radioactive gas formed (a mixture of Rn-220 and Rn-219) in a chamber not exposed to the electric field. The gas can be kept for a relative short period ( about 1 minute) to allow natural decay of formed Rn-219. The decay of the Rn-220 will then be in the collecting vessel in the presence of the electric field and allow capture of Pb-212 in essential absence of the decay products of Rn-219. Thus, in embodiments, the method comprises maintaining the radioactive gas in absence of an electric field, preferably for a period of between 30 and 180 seconds prior to subjecting the gas to the electric field. In embodiments, the apparatus hence contains a chamber positioned between the emanation vessel (1) and the collection vessel (2), optionally via tubing, and wherein preferably the chamber is not provided with or influenced by the electric field in the collection vessel (3).

In embodiments, the source of the radioactive gas, such as the Th-228 or Ra-224 source, can be provided in a liquid and the radioactive gas such as Rn, more preferably Rn-220, can form bubbles. Bubble formation can be promoted by providing a carrier gas with a carrier gas flow. When the source of the radioactive gas is not in solution, the carrier gas will transport the radioactive gas from the emanating source. The radioactive gas can adhere to the bubble surfaces and/or enters the bubbles, can be released from the liquid and transported from the liquid by the gas flow. The smaller the bubbles and the larger the amount of bubbles, the more radioactive gas can be extracted from the fluid. There is a preference to have a relative low gas flow, and a maximum amount of bubbles. Preferably most of (more than 90%, preferably the majority) of the radioactive gas that is generated is effectively removed. It is preferred that that bubble surface is maximized to allow the radioactive gas to be taken with or within the bubbles from the fluid. This can be achieved in multiple ways, but in case the gas flow needs to minimized, maximizing the amount of bubbles (minimizing bubble size) is preferred. The bubbling principle has been described in US2018/0047474. Minimizing the carrier gas flow is preferred to limit the required volume for the collection vessel (3).

The gaseous daughter can be removed by flowing a carrier gas along a surface upon which the source material (2) of the radioactive gas, such as Th-228 and/or Ra-224 has been provided. The gaseous daughter (in the case of Th-228 and/or Ra-224 this is Rn-220) is released by diffusion and/or recoil, and taken (further) from the source surface by the gas flow.

The fluid in which the source of the radioactive gas is provided can be an aqueous fluid, a charged (ionic) fluid, an organic solvent or a mixture thereof. The organic solvent can be a polar organic solvent, such as DMF, DMSO, lower (C1-C4) alcohols. The source of the radioactive gas can be provide as a solution, dispersion or emulsion in the fluid. In preferred embodiments, the aqueous fluid is one or more selected from the group consisting of water, an aqueous buffer and an aqueous acidic solution. There is a preference for an aqueous fluid in wherein the source of the radioactive gas is dissolved, such as a solution of a Th-228 salt. It is preferred that the acid in the acidic aqueous solution is selected from the group consisting of a mineral and an organic acid, preferably selected from the group consisting of hydrochloric acid, nitric acid, phosphoric acid, sulfuric acid, acetic acid, citric acid and mixtures thereof. There is a preference for a mineral acid, more preferably hydrochloric acid. To provide adequate dissolution of the source of the radioactive gas, like Th-228 or Ra-224 , the fluid preferably has an acidic pH, such as a pH of < 7, preferably < 6, more preferably below 4. Alternative complexing agents and ligands can be used that are suitable for the complexation of dissolved ions of the source of the radioactive gas, like Th-228 or Ra-224 ions, such as EDTA and other nitrogen containing macrocycles or crown ethers.

In embodiments, the thorium radionuclide can be Th-232, Th-230,Th-228, Th-227, preferably Th-228. The thorium radionuclide may also be provide as the uranium parent, U-232. In embodiments, the radium radionuclide can be Ra-226, Ra-224, Ra-223, preferably Ra-224. In embodiments, the radon radionuclide can be Rn-222, Rn-220, Rn-219, preferably Rn-220.

In the embodiment aimed at the generation of Pb-212 and compared to conventional Pb-212 generation in a column set up, the fluid or solid source in the emanation section which contains the U-232/Th-228/Ra-224 radioactive gas source can be exposed to a carrier gas flow (the gas flow allows removing gaseous daughters from the source from which radon emanates) thereby extracting gaseous Rn-220 from the source. The gaseous Rn-220 after decay the polonium and lead charged daughter radionuclides (16) are repelled by the positive electrode and attracted to the negative pole of the electric field. By proving the negative pole in solution (for instance by immersion of the electrode within the liquid), the polonium and lead ions are captured in solution directly and not on the electrode surface. When enough material is captured, the lead can be retrieved by removing the liquid in which the negative pole is immersed directly. The amount of dissolution liquid is not critical, as long as the whole negative pole is at least covered by the liquid. The radionuclide concentration can be tailored to need by using more or less liquid. The method of the present invention has at least similar efficiency in Pb-212 retrieval as a column set up, but avoids the use of columns, thereby limiting waste streams, elution losses and avoiding column deterioration by radiation damage. The resulting charged daughter radionuclides (16) in embodiments of the method of the invention are Pb-214, Pb-212, Pb-211 and/or Bi-214, Bi-212, Bi-211, preferably Pb-214, Pb-212 and Pb-211, more preferably Pb-212.

The liquid (7A) that is used for the capture of the daughter radionuclides (16) is preferably a liquid (7A) that is capable of capturing the daughter radionuclides (16) without decomposition of the liquid (7A) as a consequence of the radioactive decay of the radionuclides produced. The liquid (7A) can be a liquid that allows for the easy and convenient further handing of the daughter radionuclides (16).

Thus, in embodiments the liquid (7A) is an aqueous liquid, a charged (ionic) liquid, an organic solvent or a mixture thereof. The organic solvent can be a polar organic solvent, such as DMF, DMSO, lower (C1-C4) alcohols. In preferred embodiments, the aqueous liquid is one or more selected from the group consisting of water, an aqueous buffer and an aqueous acidic solution. By using an aqueous liquid, the charged daughter radionuclides, such as Pb-212, are directly available for further processing such as for target alpha therapy (TAT). The presence in the liquid of salts, such as sodium chloride, may aid in the function of the liquid as an electrically conductive solution, attracting the charged radionuclides and capable of capturing the charged daughter radionuclides. the use of a physiologically acceptable salt solution allows the direct further processing the captured charged daughter radionuclide.

It is preferred that the acid in the acidic aqueous solution is selected from the group consisting of a mineral and an organic acid, preferably selected from the group consisting of hydrochloric acid, nitric acid, phosphoric acid, sulfuric acid, acetic acid, citric acid and mixtures thereof. There is a preference for a mineral acid, more preferably hydrochloric acid. To provide adequate dissolution of the charged daughter radionuclides, the liquid (7A) preferably has and acidic pH, such as a pH of < 7, preferably < 6, more preferably below 4. Alternative complexing agents and ligands can be used that are suitable for the complexation of charged radionuclides such as EDTA and other nitrogen containing macrocycles or crown ethers.

The emanated radioactive gas from the source material (2) can be transported using a carrier gas. The use of a carrier gas is advantageous as it transport the emanated gas away from the source. As outlined elsewhere, alpha decay, as in the case of the Th-228/Ra-228/Rn-220/Po-216/Pb-212 pathway has as one of its characteristics the presence of a recoil effect. The recoil effect can cause that the formed radionuclides when become embedded in surrounding solid material from which they are difficult to remove. The carrier gas aids in transporting the formed radioactive gas away from the source.

The carrier gas is preferably an inert gas and/or a noble gas or a gas that is not affected by radioactive decay of the radionuclides present. Thus the gas may also be air, oxygen or, carbon dioxide, as long it remains unaffected by radioactive decay. Thus, the carrier gas can be selected from the group consisting of nitrogen, helium, argon, carbon dioxide, air and mixtures thereof. There is a preference for nitrogen and/or helium or mixtures thereof.

The flow of the carrier may vary and can be determined depending on the circumstances. The flow is preferably high enough to transport the radioactive gas away from the source material (2).

The flow of the radioactive gas and optional carrier gas should be enough to avoid loss by decay, by neutralization or by embedding in solid materials by decay and recoil underway. The gas flow should be adequate to transport the radioactive gas that is formed and liberated from the source material (2) over a distance such that any radioactive decay involving alpha particles does not leave the daughter radionuclides (16) embedded in the source material due to a recoil effect. The flow should be high enough to bring the radioactive gas and the charged daughter radionuclides (16) formed within the reach of the electric field such that any charged daughter radionuclides (16) are attracted towards the liquid (7A) embedded electrode . The gas flow should be preferably chosen in a ratio to the total system volume per minute of 1:1 to 1:25. For example, if the total system volume is 250 mL, the gas flow should be preferably chosen in the range of 10-250 mL/min.

In the radioactive decay chain of the Th-228/Ra-224 source material, Rn-220 is emanated as a radioactive gas and can be removed from the surface of the source material (2) by the gas flow. The Rn-220 emits an alpha particle, resulting in Po-216 ion as a charged radioactive daughter radionuclide with a very short half-life. The Po-216 emits also an alpha particle, resulting in Pb-212 as a charged radioactive daughter radionuclide. Both charged radioactive daughter radionuclide Po-216 and Pb- 212 can be influenced by the applied electric field between the electrodes (5,6).

The electric field is provided between two electrodes (5,6) (a positive electrode and a negative electrode). Each electrode can contain one or more multiple electrodes. The electric field is maintained between the electrodes (5,6) by isolating the two electrodes (5,6) from each other such that no current flows. The electric field is maintained to capture any charged daughter radionuclides (16).

The charged daughter radionuclides (16) are subject to an electric field between the two electrodes (5,6). The electric field between the electrodes (5,6) will attract the charged daughter radionuclides (16) to the electrode that has the opposite charge of the charged daughter radionuclide. The charged daughter radionuclides (16) will be repelled by the electrode that carries the opposite charge. The efficiency of attracting the charged daughter radionuclides (16) to the electrode will depend, among others, on the strength of the electric field. The electric field strength between the electrodes (5,6) is such that the charged daughter radionuclides (16) are attracted to the electrode. The strength of the electric field also depends on the medium, the dielectric. The voltages over the electrodes (5,6) is typically between 10 and 10.000 volt, between 100 and 7500 volt, between 500 and 5000 volt. This provides an adequate electric field.

In one embodiment, the gas is contacted with a liquid, wherein the liquid (7A) carries an electric charge, preferably a negative charge. The negative charge can be provided to the liquid (7A) by providing an electric field. By providing the liquid (7A) with an electric charge, the oppositely charged daughter radionuclides (16) are attracted to the liquid (7A) and captured therein. In the embodiments aimed at the capture of positively charged daughter radionuclides (16) (such as Po-216, Pb-212), the liquid (7A) is negative charged.

The method of the invention can be performed at a reduced pressure (from 0.01 up to 1 bar), ambient pressure (about 1 bar) or increased pressure (more than 1 bar). The method of the invention can be performed at a temperature between 5 and 100 degrees Celsius, more preferably between 10 and 90 degrees Celsius. It is considered advantageous to use a temperature above ambient in the gas emanating source to remove the radioactive gas from the gas emanating source.

The electric field between the electrodes (5,6) causes the charged radioactive daughter radionuclides (16) to be absorbed in the liquid. This provides a solution of the charged radioactive daughter radionuclides (16) in the liquid (7A). To this end, the liquid (7A) is placed between the (electric field of the) electrodes (5,6). The electric field will attract the charged radioactive daughter radionuclides (16) towards one of the electrodes (5,6) and repel them from the other electrode (5,6). The suitable selection of the charge of the electrode (positive or negative) and the positioning of the liquid (7A) between the electrodes (5,6) cause the charged radioactive daughter radionuclides (16) to be captured in the liquid (7A) (positive ions to the negative electrode and negative ions to the positive electrode). By placing the ion attracting electrode in, under, immersed in, surrounded by or submerged in the liquid, the charged daughter radionuclides (16) that are attracted to the electrode are captured in the liquid (7A). This capturing step of the process provides the radioactive daughter ions directly in the liquid (7A) and limits or prevents the radionuclides being deposited directly on the electrode surface.. The liquid (7A) can be isolated, used as is, or subject to furthers steps such as purification, complexation and use as a radiochemical material for further labelling or synthesis of radiopharmaceutical end products for in targeted radiotherapy.

In a preferred embodiment, the invention pertains to a method for the generation of Pb-212 radionuclides comprising the steps of
a. providing a Rn-220 gas,
b. allowing the radioactive gas to decay to form charged daughter radionuclides (16) comprising Po-216 and/or Pb-212 ,
c. providing a liquid (7A) in contact with the radioactive gas,
d. subjecting the charged daughter radionuclides (16) to an electric field between two electrodes (5,6) thereby absorbing the Po-216 and/or Pb-212 charged daughter radionuclides (16) in the liquid, and
e. isolating the liquid (7A) comprising the charged daughter radionuclides.

In the method for the generation of Pb-212, the Rn-220 gas decays into Po-216 as the charged daughter radionuclide by the emission of an alpha particle. The Po-216 will, due to electric field move towards the oppositely charged electrode in a matter of milliseconds. The Po-216 will subsequently decay in to Pb-212, preferable and predominantly in the liquid (7A).

In a further aspect, the invention pertains to an apparatus for the preparation and isolation of radionuclides comprising an emanation vessel (1) and a collection vessel (3), wherein the emanation vessel is connected to the collection vessel (3) or wherein the emanation vessel (1) and collection vessel (3) are comprised in a singular vessel,
a. wherein the emanation vessel (1) comprises
   i. a radioactive gas emanating source material (2),
   ii. optionally, a gas inlet (4),
b. wherein the collection vessel (3) contains
   i. a first electrode (5) connected to an electric source (8),
   ii. a second electrode (6) connected to the electric source (8),
   iii. a liquid section (7),
   iv. optionally, a gas outlet (11).

In the apparatus of the invention, the radioactive gas emanating source (2) can be a radon emanating source. The apparatus (the generator of charged radionuclides) of the invention can be manufactured out of radiation resistant and inert materials, that can withstand the conditions in the generator for a long time, and can therefore accommodate the parent radionuclides such as U-232/Th-228/Ra-224.

The emanation vessel (1) comprising the radioactive gas emanating source material (2) can be provided in the form of a container capable of containing a fluid comprising the radioactive gas emanating source (2) or in a form that allows the radioactive gas emanating source (2) to be provided as a single (point) source element, but it is preferred in embodiments to provide the source as a solution, a powder, a foam, a foil, a sponge, a salt or on a carrier. The source material (2) may be provided in elemental form or as a compound, such as a salt or oxide. The emanation vessel (1) may further be provided with a gas inlet (4). The gas inlet (4) can be used for the provision of a carrier gas to transport the emanated radioactive gas. The emanation vessel (1) can be removably connected to the collection vessel (3) to allow exchange of radioactive gas emanating source material (2), for instance when the source material (2) is depleted. A separate gas supply may be provided, connected to the gas inlet (4).

The emanation vessel (1) can be removably connected to the collection vessel (3). The emanation vessel (1) can be in direct contact with the collection vessel (3) or via a connecting element (15) such as a connection tube. The emanation vessel (1) is connected to the collection vessel (3) at one end of the collection vessel (3). The connecting element (15) may also function as a gas outlet of the emanation vessel (1) to transport the gas from the emanation vessel (1) to the collection vessel (3). The emanation vessel (1) and the collection vessel (3) together form a closed vessel. Gas and liquids can be provided to the closed vessel and removed from the closed vessel through provided inlets and outlets for liquid and/or gas.

The collection vessel (3) is provided with two electrodes (5) and (6) connected to a power source/voltage generator (8). The collection vessel (3) has a volume (holding section). The volume of the holding section is selected such that the radioactive gas (optionally in combination with the flow generated by a carrier gas) remains in this volume for a period sufficient to allows the radioactive gas to predominately (more than 90%) decay in to charged daughter radionuclides. In embodiments, the holding section is capable of collecting the radioactive gas for a time sufficient to yield charged daughter radionuclides. For instance in the case of radion-220, with a half time of 51 seconds, the preference collection time is about 5 minutes. Typically the preferred collection time is from one to ten, preferably from about three to seven times the halftime of the radioactive gas. The preferred collection time is a compromise between:
- When the required residence time is too-long the collection vessel (3) must be too large or the flow through the emanation vessel is too small.
- When the required residence time is too-short a significant fraction of the Rn-220 will decay outside the collection vessel (3) and thereby not contribute to the yield.

Between the two electrodes (5,6) an electric potential difference can be provided, creating an electric field. The two electrodes (5,6) are electrical isolated from each other through electric isolation (12). The inner wall of the collection vessel (3) can form one of the electrodes (5) . On the other distal end of collection vessel (3) and removed from the end connected to the emanating vessel (1), the other electrode (6) can be provided. The polarity of the electrodes (5,6) can be selected by the power source/voltage generator (8). Electrode (6) is provided in a liquid (7A) or is located under the liquid (7A). The liquid (7A) is preferably located at the bottom end of the collection vessel (3) . The section of the collection vessel (3) that contains the liquid (7A) can be depicted as the liquid section (7). The liquid section (7) contains the liquid (7A) . The liquid (7A) is at least partially within the electric field between the two electrodes (5,6). The electrode (6) is preferably immersed in the liquid (7A) or is located in a separate section below the liquid, such as encompassed or incorporated in the bottom of the vessel in such a way that the electric field between the electrodes (5,6) passes through the liquid (7A) and is capable of exerting an electric force on the charged radioactive daughter radionuclides.

A gas outlet (11) is connected to the collection vessel (3) . The gas outlet (11) is located near the surface of the liquid (7A) in the collection vessel (3) such that the gas flow from the emanation source flows through the collection vessel (3) to the gas outlet (11).

The carrier gas can transport the radioactive gas form the emanation vessel (1) through the collection vessel (3). Through natural radioactive decay, the radioactive gas will form radioactive charged daughter radionuclides. The charged daughter radionuclides, in the preferred case of Rn-220, charged daughter radionuclides (16) Po-216 and Pb-212 are formed, are repelled by electrode (5) encompassing the gas volume and are attracted by the negative electrode (6) and will be absorbed by the liquid (7A). The radioactive charged daughter radionuclides (16) formed can be isolated by removal from the liquid section (7). Removal and /or exchange from the liquid section (7) can be achieved by providing the liquid section (7) with a liquid inlet (9) and/or a liquid outlet (10). The gas flow can exit the collection vessel (3) through the gas outlet (11). The gas outlet (11) can be placed above the surface of liquid, although in embodiments, the exiting gas can be transported or bubbled through the liquid (7A). The exited gas may be filtered from undesired particles and compounds through filter (13) and emitted through gas vent (14). Alternatively, the gas may be recirculated by providing a connection between the gas outlet (11) and the gas inlet (4). The removed fluid contains the liquid comprising the radioactive daughter ions.

In embodiments, the radioactive gas emanating source can be a thorium or radium radionuclide. The emanation vessel (1) can be removably connected to the collection vessel (3) to allow exchange of radioactive gas emanating source material (2), for instance when the source material (2) is depleted. Alternatively the emanation vessel (1) contains an insertable module containing the radioactive source material (2) to allow exchange of the source material (2).

In embodiments, the collection vessel (3) , preferably the liquid section (7) comprises a liquid outlet and/or liquid inlet. The presence of a liquid (7A) in and/or outlet allows for the exchange and/ or removal of the liquid (7A) contain the formed charged daughter radionuclides (16) without disassembly of the apparatus.

In embodiments, the electrode (6) is placed outside the liquid section (7) of the collection vessel (3) . In this embodiment, the electrode placed outside the collection vessel (3) can for instance be placed directly underneath the liquid section (7) or incorporated/embedded in the material of the liquid section (7). The material of the liquid section (7) is preferable a solid material having a relative low electric permittivity, allowing the electric field to exert an attracting force on charged daughter radionuclides. Examples are plastics, glass or composites.

In embodiments, the electrode (6) is placed in the liquid (7A) in the liquid section (7). Preferably, the electrode (6) is positioned in the liquid section (7) such that the electrode (6) is immersed in the liquid (7A). It is preferred that in certain embodiments, the electrode (6) is separated from the electrode (5) by the liquid (7A). Preferably, the electrode (6) is isolated from/ is not in electrical contact with electrode (5), using electric isolation (12) . In embodiments, the collection vessel (3) comprises a holding section connected to the liquid section (7). The holding section (18) serves to hold the gas for a period long enough for the charged daughter radionuclides (16) to form through decay of the radioactive gas. The holding section (18) is placed between the emanation section (or the connection tube) and the liquid section (7). The holding section (18) connected to the liquid section (7) may be electrically isolated from the liquid section (7).

The two electrodes (5, 6) are preferably connected to allow a voltage to be applied over the electrode. The voltage can be provided by a voltage generator (8) in contact with the electrodes (5,6).

In embodiments, the holding section (18) or the liquid section (7) contains a gas outlet (11).

In embodiments, the electrode (5) is a conductive material significantly encompassing the holding section (18). In preferred embodiments, the holding section (18) is a conductive material. In embodiments, the electrode (6) is an electrical conductive material such as a noble metal, Pt, W, stainless steel, alloys, carbon, copper, aluminum. In embodiments where the electrode is in direct contact with the liquid, the choice of materials is preferably inert to the liquid (such as an acidic solution) like noble metals, Pt, W, stainless steel, alloys, carbon etc. In embodiments, the gas outlet (11) contains a filter such as a carbon or HEPA filter.

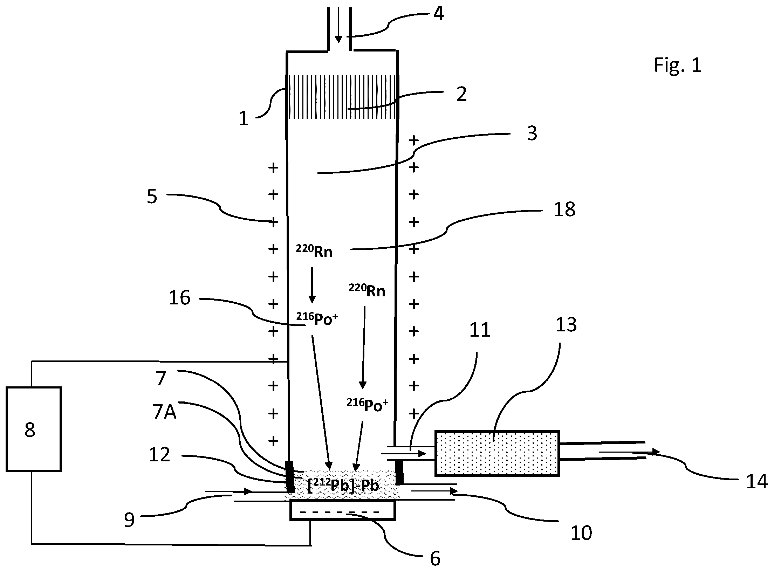

In one embodiment as illustrated in **Fig** 1, the emanation vessel (1) and collection vessel (3) can be combined in one body, for instance with the radioactive source on top through which a carrier gas can carry the radioactive gas to the collection vessel (3) . The wall of the collection vessel (3) and in particular the holding section (18), is connected to the power source and functions as the electrode (5), and set at an electrical charge to preventing deposition of the formed charged daughters. The liquid (7A) is provided in the liquid section (7) at a position where the electrode (6) (charged opposite to the charged daughter radionucleotides), that is connected to the power source, is immersed in the liquid (7A) and/or wherein the liquid (7A) encompasses the electrode or is placed between the electrodes (5,6) in such a way that the electric field generated between the electrodes (5,6) forces the charged daughters radionuclides to move and be absorbed by the liquid (7A). By the electric field between the electrodes (5,6), the charged daughters radionuclides (in the case of the Rn-220 to Pb-212 decay chain positively charged) are attracted to the (in the case of the Rn-220 to Pb-212 negatively charged) electrode and are subsequently captured and absorbed in the liquid (7A) in the liquid section (7).

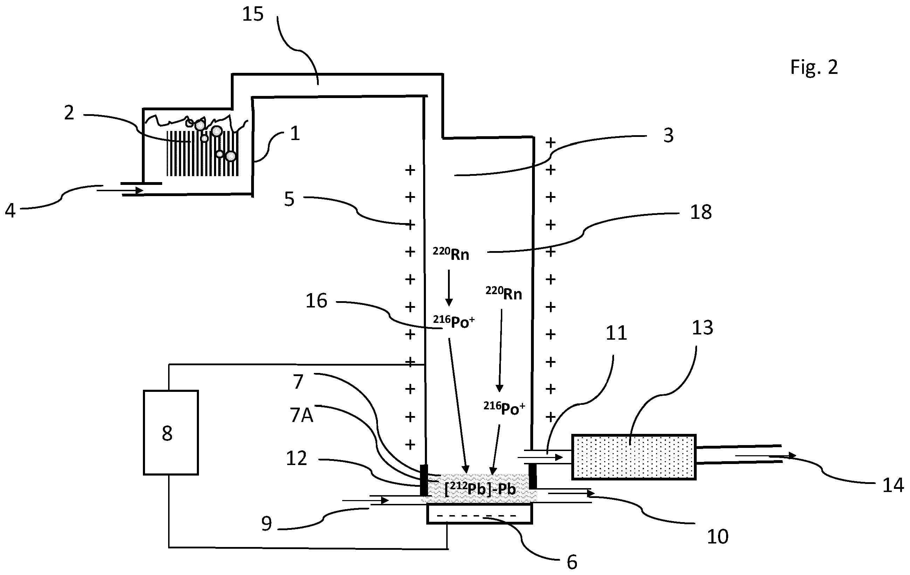

In one embodiment as illustrated in **Fig 2****,** the emanation vessel (1) and collection vessel (3) can be two separate bodies, connected though a connection tube (15). The emanation vessel (1) contains a liquid comprising the radioactive source, such as Th-228 and/or Ra-224. In this embodiment the radioactive source can be provided as a solution which avoids recoil of the daughter radionuclide in solid source material. The emanated gas can bubble out of the liquid, optionally aided by bubbling a carrier gas via a gas inlet (4) through the liquid containing the radioactive source material (2) such that the radioactive gas is carried along to the collection vessel (3) .

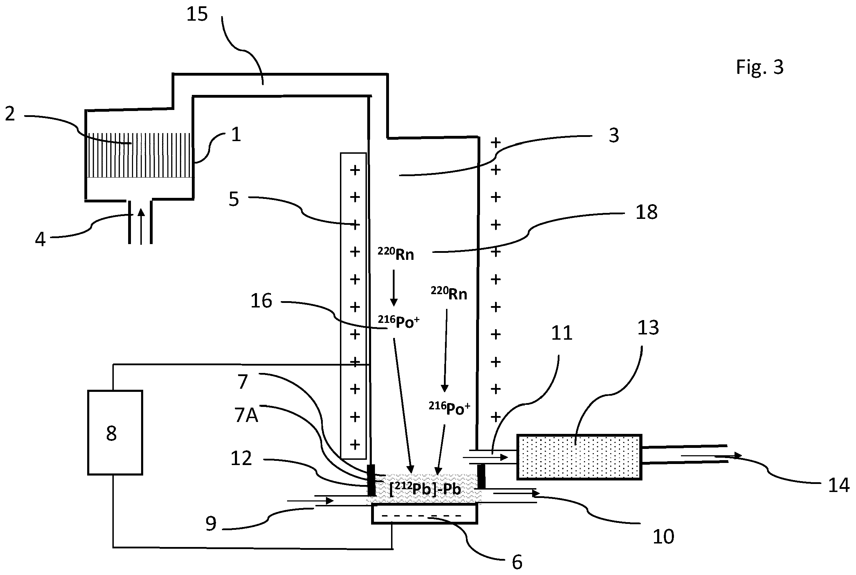

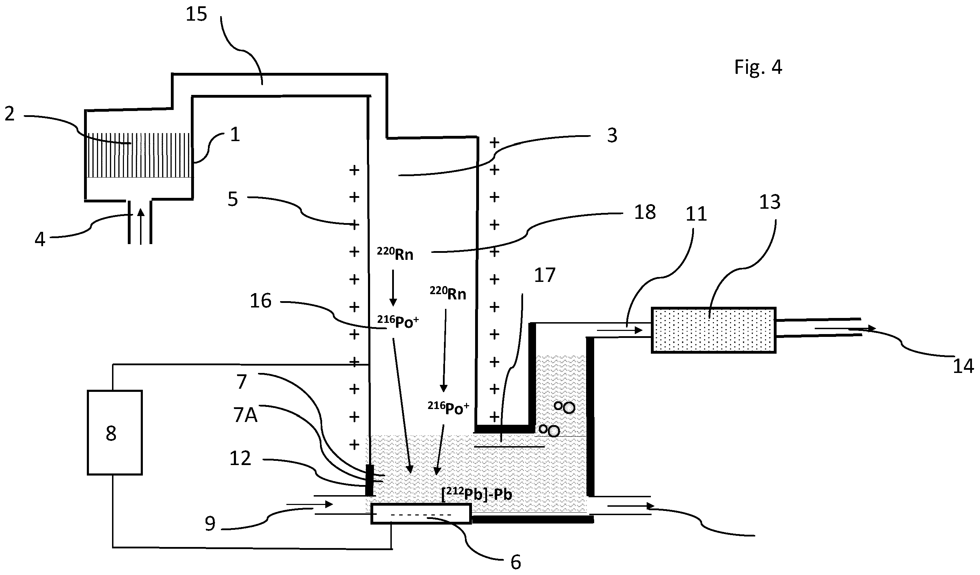

In one embodiment as illustrated in **Fig 3****,** the emanation vessel (1) and collection vessel (3) can be two separate bodies, connected though a connection tube (15). The emanation vessel (1) contains a solid comprising the radioactive source, such as Th-228 and/or Ra-224. In this embodiment, the radioactive source can be provided in the form of thin layers or a sponge like material to avoid recoil of the daughter radionuclide in solid source material (2), and/or a material with large surface area upon which the parent radionuclide is deposited in a thin layer. The emanated gas can move away from the source material (2) via diffusion, optionally aided by providing a carrier gas via a gas inlet (4) along the solid containing the radioactive source material (2) such that the radioactive gas is carried along to the collection vessel (3) . In one embodiment as illustrated in **Fig 4****,** the liquid section (7) can be configured as having two (tubular) legs connected by a (tubular) bend, for instance in a U-type construction. The two connected legs contain a liquid (7A) . The equilibrium level of the liquid (7A) when there is no gas flow can be above the bend. The liquid surface in one leg is in contact with the gas in the collection vessel (3) . When pressure builds in the leg connected to the collection vessel (3) , the level of the liquid (7A) at that side will lower. A connection tube or gas-pass-through (17) can be provided to allow the excess gas to transfer to the other leg. The excess gas can bubble through the liquid (7A) and improve the removal of any ions from the gas prior to exit through the gas vent (14).

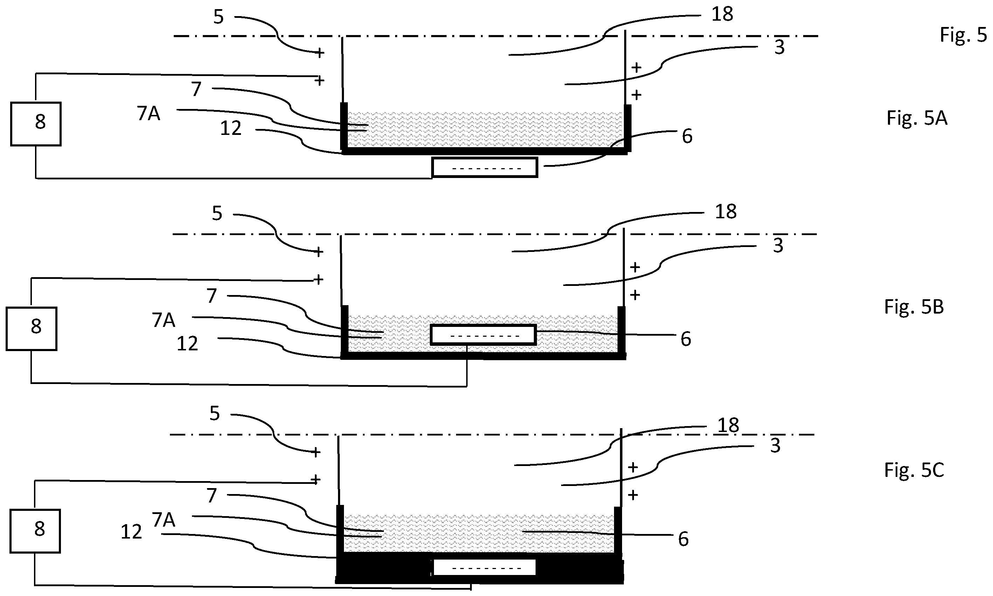

In one embodiment as illustrated in **Fig 5****,** the positioning of electrode (6) is illustrated as below and outside the liquid section (7) **(****Fig 5A****),** inside the liquid section (7) **(****Fig 5B****),** and embedded in the material of the liquid section (7) **(****Fig 5C****),**

### Examples

### Pb-212 Generator Experimental Setup

### Equipment

The radioactivity amounts of the various radionuclides is measured using an high purity germanium detector.

The experimental setup consists of an gas flow controller connected to a 25 mL emanation vial. The emanation vial is connected to a 400 mL collection vessel, which contains a gas inlet, gas outlet, liquid inlet/outlet, and a platinum electrode connected to the negative pole of the high voltage power supply (0 - 5 kV). A layer of copper foil is attached to the inside walls of the collection vessel, which is connected to the positive pole of the power supply. The gas outlet of the collection vessel is connected to a trapping column filled with zeolite molecular sieves.

### Reagents

An aqueous solution of [Th-228]ThCl₄ obtained from NRG. All other reagents (0.1M HCl,) is acquired at Sigma Aldrich. Nitrogen gas is used as carrier gas.

### Measurement of Pb-212 radioactivity

The radioactivity of Pb-212 is measured by gamma-spectroscopy, based on the distinct 2.6 MeV gamma photon energy of TI-208, a short lived daughter of Pb-212. Due to the gamma energy being high, self-shielding and shielding by other materials can be neglected, providing quantitative measurement of the TI-208 and therefore also the Pb-212 radioactivity. The measurement can be quantitative largely irrespective of source geometry, by measuring at adequate distance facilitated by the high energy of the gamma to be measured, rendering a point source.

### Determining Th-228 radioactivity of the Th-228 source

The amount of Th-228 in the Th-228 source is measured by gamma-spectroscopy measurement of the formed Pb-212, either after full ingrowth of the Pb-212, or by measuring in intervals and determining the ingrowth over time.

### Determining Pb-212 production capability of Th-228 source

The production capability of Pb-212 generated from the Th-228 source in the emanation vessel is determined by leading the carrier gas containing the Rn-220 through multiple trapping columns (zeolite). This column is of a size, which in combination with the gas flow, will trap any radon species and daughters in the gas stream, additional columns are added in series to confirm all Radon ad Radon-daughters are trapped and to ensure no Radon escapes to the environment. The trapped Rn-220 decays to Pb-212 on the trapping column. Measuring the activity of Pb-212 on the trapping column by gamma-spectroscopy provides a (relative) quantification of the amount of Rn-220 / Pb-212 that is retrieved from the Th-228 source in the emanation vessel. This exercise is regularly repeated to ensure the Radon released from the source is stable and unchanged over the course of experiments.

### Determine efficiency of trapping Pb-212 in 0.1M HCl using a submerged electrode

A solution of [Th-228]ThCl₄ (5 mL, 50 MBq) is added to the emanation vial. The collection liquid is subsequently added to the collection vessel, with a volume large enough to fully submerge the negative electrode. A voltage of 3.5 kV is applied between the two electrodes, and a nitrogen flow of 50 mL/min is bubbled through the [Th-228]ThCl₄ solution and led through the collection chamber towards column for different durations ranging from a few hours up to 24 hours. Subsequently, the gas flow and power supply is turned off, and the collection liquid is collected from the collection vessel.

The trapping efficiency is determined by measuring the amount of Pb-212 collected in the collection liquid and comparing this to the previously determined production capability of the Th-228 source. The amount of Pb-212 collected on the trapping column filled with molecular sieves is measured to determine the amount of Pb-212 which emanated from the Th-228 source, but has not been collected in the collection liquid. The total amount measured is added up to what is expected to emanate from the source.
- 1.: Emanation vessel
- 2.: Source material
- 3.: Collection vessel
- 4.: Gas inlet
- 5.: Electrode
- 6.: Electrode
- 7.: Liquid section
- 7A.: Liquid
- 8.: Power source
- 9.: Liquid inlet
- 10.: Liquid outlet
- 11.: Gas outlet
- 12.: Electric insulation
- 13.: Filter
- 14.: Gas vent
- 15.: Connection tube
- 16.: Charged daughter radionuclides
- 17.: Gas pass through
- 18.: Holding section

## Claims

1. Method for the generation and isolation of radionuclides comprising the steps of
a. providing a radioactive gas,
b. allowing the radioactive gas to decay to form charged daughter radionuclides,
c. providing a liquid in contact with the gas,
d. subjecting the charged daughter radionuclides (16) to an electric field between two electrodes (5,6), thereby absorbing the charged daughter radionuclides (16) in the liquid, and
e. isolating the liquid comprising the charged daughter radionuclides (16).

2. Method according to claim 1, wherein the radioactive gas is provided in a gas flow with a carrier gas.

3. Method according to claims 1 or 2 wherein the daughter radionuclides (16) are Pb-214, Pb-212, Pb-211 and/or Bi-214, Bi-212, Bi-211, preferably Pb-212.

4. Method according to claims 1-3, wherein the liquid is an aqueous liquid and has a pH of < 7, preferably < 6, more preferably below 4.

5. Apparatus for the preparation and isolation of radionuclides comprising an emanation vessel and a collection vessel (3), wherein the emanation vessel is connected to the collection vessel (3) or wherein the emanation vessel and collection vessel (3) are comprised in a singular vessel,
a. wherein the emanation vessel comprises
i. a radioactive gas emanating source,
ii. optionally a gas inlet,
b. wherein the collection vessel (3) contains
i. an electrode (5) connected to an electric source,
ii. an electrode (6) connected to the electric source,
iii. a liquid section (7),
iv. optionally a gas outlet (5).

6. Apparatus according to claim 5, wherein electrode (5) is placed outside the liquid section (7).

7. Apparatus according to claim 5 or 6, wherein the electrode (6) is placed in the liquid section (7).

8. Apparatus according to claims 5-7, wherein the electrode (6) is positioned in the liquid section (7) such that the electrode (6) is immersed in the liquid (7A).

9. Apparatus according to claims 5-8, wherein the electrode (6) is separated from the electrode (5) by the liquid (7A).

10. Apparatus according to claims 5-9, wherein the electrodes (5,6) are electrically isolated from each other.

11. Apparatus according to claims 5-10, wherein the electrode (5) is isolated from/ is not in electrical contact with electrode (6).

12. Apparatus according to claims 5-11, wherein the collection vessel (3) contains a holding section connected to the liquid section (7).

13. Apparatus according to claims 5-12, wherein the holding section is capable of collection the radioactive gas for a time sufficient to yield charged daughter radionuclides (16).

14. Apparatus according to claims 5-13, wherein the electrode (6) is provided in, near or behind the liquid (7A).

15. Apparatus according to claims 5-14, wherein the collection vessel (3) or the holding section contains a gas outlet (11).

## Patentansprüche

1. Verfahren zum Erzeugen und Isolieren von Radionukliden, umfassend die folgenden Schritte:
a. Bereitstellen eines radioaktiven Gases,
b. Erlauben, dass das radioaktive Gas zerfällt, um geladene Tochterradionuklide zu bilden,
c. Bereitstellen einer Flüssigkeit in Kontakt mit dem Gas,
d. Aussetzen der geladenen Tochterradionuklide (16) gegenüber einem elektrischen Feld zwischen zwei Elektroden (5,6), wodurch die geladenen Tochterradionuklide (16) in der Flüssigkeit absorbiert werden, und
e. Isolieren der Flüssigkeit, die die geladenen Tochterradionuklide (16) umfasst.

2. Verfahren nach Anspruch 1, wobei das radioaktive Gas in einem Gasfluss mit einem Trägergas bereitgestellt wird.

3. Verfahren nach einem der Ansprüche 1 oder 2, wobei die Tochterradionuklide (16) Pb-214, Pb-212, Pb-211 und/oder Bi-214, Bi-212, Bi-211, vorzugsweise Pb-212, sind.

4. Verfahren nach einem der Ansprüche 1-3, wobei die Flüssigkeit eine wässrige Flüssigkeit ist und einen pH-Wert von < 7, vorzugsweise < 6, besonders bevorzugt unter 4, aufweist.

5. Vorrichtung zum Herstellen und Isolieren von Radionukliden, umfassend ein Emanationsgefäß und ein Sammelgefäß (3), wobei das Emanationsgefäß mit dem Sammelgefäß (3) verbunden ist oder wobei das Emanationsgefäß und das Sammelgefäß (3) in einem einzigen Gefäß bestehen,
a. wobei das Emanationsgefäß Folgendes umfasst:
i. eine radioaktives Gas emanierende Quelle,
ii. gegebenenfalls einen Gaseinlass,
b. wobei das Sammelgefäß (3) Folgendes enthält:
i. eine Elektrode (5), die mit einer elektrischen Quelle verbunden ist,
ii. eine Elektrode (6), die mit der elektrischen Quelle verbunden ist,
iii. einen Flüssigkeitsabschnitt (7),
iv. gegebenenfalls einen Gasauslass (5).

6. Vorrichtung nach Anspruch 5, wobei die Elektrode (5) außerhalb des Flüssigkeitsabschnitts (7) angeordnet ist.

7. Vorrichtung nach Anspruch 5 oder 6, wobei die Elektrode (6) in dem Flüssigkeitsabschnitt (7) angeordnet ist.

8. Vorrichtung nach einem der Ansprüche 5-7, wobei die Elektrode (6) derart in dem Flüssigkeitsabschnitt (7) angeordnet ist, dass die Elektrode (6) in die Flüssigkeit (7A) eingetaucht ist.

9. Vorrichtung nach einem der Ansprüche 5-8, wobei die Elektrode (6) durch die Flüssigkeit (7A) von der Elektrode (5) getrennt ist.

10. Vorrichtung nach einem der Ansprüche 5-9, wobei die Elektroden (5,6) elektrisch voneinander isoliert sind.

11. Vorrichtung nach einem der Ansprüche 5-10, wobei die Elektrode (5) von der Elektrode (6) isoliert ist / sich nicht in elektrischem Kontakt damit befindet.

12. Vorrichtung nach einem der Ansprüche 5-11, wobei das Sammelgefäß (3) einen Halteabschnitt enthält, der mit dem Flüssigkeitsabschnitt (7) verbunden ist.

13. Vorrichtung nach einem der Ansprüche 5-12, wobei der Halteabschnitt zur Sammlung des radioaktiven Gases für eine Zeit in der Lage ist, die ausreicht, um geladene Tochterradionuklide (16) hervorzubringen.

14. Vorrichtung nach einem der Ansprüche 5-13, wobei die Elektrode (6) in, nahe oder hinter der Flüssigkeit (7A) bereitgestellt ist.

15. Vorrichtung nach einem der Ansprüche 5-14, wobei das Sammelgefäß (3) oder der Halteabschnitt einen Gasauslass (11) enthält.

## Revendications

1. Procédé destiné à générer et isoler des radionucléides comprenant les étapes suivantes :
a. fourniture d'un gaz radioactif,
b. décroissance naturelle du gaz radioactif pour former des radionucléides de filiation chargés,
c. fourniture d'un liquide en contact avec le gaz,
d. soumission des radionucléides de filiation chargés (16) à un champ électrique entre deux électrodes (5, 6), pour absorber ainsi les radionucléides de filiation chargés (16) dans le liquide, et
e. isolement du liquide comprenant les radionucléides de filiation chargés (16).

2. Procédé selon la revendication 1, dans lequel le gaz radioactif est fourni dans un écoulement gazeux avec un gaz vecteur.

3. Procédé selon les revendications 1 et 2 dans lequel les radionucléides de filiation (16) sont Pb-214, Pb-212, Pb-211 et/ou Bi-214, Bi-212, Bi-211, de préférence Pb-212.

4. Procédé selon les revendications 1 à 3, dans lequel le liquide est un liquide aqueux et a un pH < 7, de préférence < 6, mieux encore inférieur à 4.

5. Appareil destiné à préparer et isoler des radionucléides comprenant une cuve d'émanation et une cuve de collecte (3), la cuve d'émanation étant raccordée à la cuve de collecte (3), ou bien la cuve d'émanation et la cuve de collecte (3) étant comprises dans une seule cuve,
a. dans lequel la cuve d'émanation comprend
i. une source d'émanation de gaz radioactif,
ii. éventuellement une entrée de gaz,
b. dans lequel la cuve de collecte (3) contient
i. une électrode (5) reliée à une source électrique,
ii. une électrode (6) reliée à la source électrique,
iii. une section liquide (7),
iv. éventuellement une sortie de gaz (5).

6. Appareil selon la revendication 5, dans lequel l'électrode (5) est placée à l'extérieur de la section liquide (7).

7. Appareil selon la revendication 5 ou 6, dans lequel l'électrode (6) est placée dans la section liquide (7).

8. Appareil selon les revendications 5 à 7, dans lequel l'électrode (6) est positionnée dans la section liquide (7) de telle sorte que l'électrode (6) est plongée dans le liquide (7A).

9. Appareil selon les revendications 5 à 8, dans lequel l'électrode (6) est séparée de l'électrode (5) par le liquide (7A).

10. Appareil selon les revendications 5 à 9, dans lequel les électrodes (5, 6) sont isolées électriquement l'une de l'autre.

11. Appareil selon les revendications 5 à 10, dans lequel l'électrode (5) est isolée de/n'est pas en contact électrique avec l'électrode (6).

12. Appareil selon les revendications 5 à 11, dans lequel la cuve de collecte (3) contient une section de retenue raccordée à la section liquide (7).

13. Appareil selon les revendications 5 à 12, dans lequel la section de retenue peut collecter le gaz radioactif pendant un temps suffisant pour produire des radionucléides de filiation chargés (16).

14. Appareil selon les revendications 5 à 13, dans lequel l'électrode (6) est disposée dans, à proximité du ou derrière le liquide (7A).

15. Appareil selon les revendications 5 à 14, dans lequel la cuve de collecte (3) ou la section de retenue contient une sortie de gaz (11).
